# EUROPEAN PATENT SPECIFICATION

(11) **EP 1 902 725 B1**
(45) Date of publication and mention of the grant of the patent: **26.03.2014**
(21) Application number: 07021431.7
(22) Date of filing: 24.02.2006
(51) Int. Cl.: A61K 38/53, A61P 27/02

(54) **Revascularization of ischemic retinal tissue**
Revaskularisierung ischämischen Netzhautgewebes
Revascularisation de tissu rétinal ischémique

(30) Priority: 24.02.2005 US 655732 P
(43) Date of publication of application: 26.03.2008
(62) Divisional of application: 06735886.1
(73) Proprietor: THE SCRIPPS RESEARCH INSTITUTE, La Jolla, CA 92037 (US)
(72) Inventor: Friedlander, Martin, Del Mar, California 92014 (US); Banin, Eyal, La Jolla, California 92037 (US); Aguilar, Edith, San Diego, California 92126 (US)
(74) Representative: Schnappauf, Georg

(56) References cited:
- WO-A-02/067970
- WO-A-03/009813

## Description

### STATEMENT OF GOVERNMENT INTEREST

A portion of the work described herein was supported by grant number EY11254 and grant number EY14174 from the National Institutes of Health. The United States Government has certain rights in this invention.

### FIELD OF THE INVENTION

This invention relates to treatment of retinal neovascular diseases. More particularly this invention relates to methods for promoting retinal revascularization by administering an angiostatic protein fragment to a patient, and to screening methods for identifying therapeutic agents to treat retinal vascular diseases.

### BACKGROUND OF THE INVENTION

Vascular diseases of the retina, including diabetic retinopathy, exudative age related macular degeneration (ARMD), retinopathy of prematurity (ROP) and vascular occlusions, are major causes of visual impairment and blindness. This group of diseases is the focus of intense research aimed to identify novel treatment modalities that will help prevent or modify pathological ocular neovascularization. For example, ARMD affects 12-15 million American over the age of 65 and causes visual loss in 10-15% of them as a direct effect of choroidal (sub-retinal) neovascularization. The leading cause of visual loss for Americans under the age of 65 is diabetes; 16 million individuals in the United States are diabetic and 40,000 per year suffer from ocular complications of the disease, often a result of retinal neovascularization. While laser photocoagulation has been effective in preventing severe visual loss in subgroups of high risk diabetic patients, the overall 10-year incidence of retinopathy remains substantially unchanged. For patients with choroidal neovascularization due to ARMD or inflammatory eye disease such as ocular histoplasmosis, photocoagulation, with few exceptions, is ineffective in preventing visual loss. While recently developed, nondestructive photodynamic therapies hold promise for temporarily reducing individual loss in patients with previously untreatable choroidal neovascularization, only 61.4% of patients treated every 3-4 months had improved or stabilized vision compared to 45.9% of the placebo-treated group.

Age related macular degeneration and diabetic retinopathy are the leading causes of visual loss in industrialized nations and do so as a result of abnormal retinal neovascularization. Since the retina consists of well-defined layers of neuronal, glial, and vascular elements, relatively small disturbances such as those seen in vascular proliferation or edema can lead to significant loss of visual function. Inherited retinal degenerations, such as retinitis pigmentosa (RP), are also associated with vascular abnormalities, such as arteriolar narrowing and vascular atrophy. While significant progress has been made in identifying factors that promote and inhibit angiogenesis, no treatment is currently available to specifically treat ocular vascular disease.

Inherited degenerations of the retina affect as many as 1 in 3500 individuals and are characterized by progressive night blindness, visual field loss, optic nerve atrophy, arteriolar attenuation, altered vascular permeability and central loss of vision often progressing to complete blindness (Heckenlively, J. R., editor, 1988; Retinitis Pigmentosa, Philadelphia: JB Lippincott Co.). Molecular genetic analysis of these diseases has identified mutations in over 110 different genes accounting for only a relatively small percentage of the known affected individuals (Humphries et al., 1992, Science 256:804-808; Farrar et al. 2002, EMBO J. 21:857-864.). Many of these mutations are associated with enzymatic and structural components of the phototransduction machinery including rhodopsin, cGMP phosphodiesterase, *rds* peripherin, and RPE65. Despite these observations, there are still no effective treatments to slow or reverse the progression of these retinal degenerative diseases. Recent advances in gene therapy have led to successful reversal of the *rds* (Ali et al. 2000, Nat. Genet. 25:306-310) and *rd* (Takahashi et al. 1999, J. Virol. 73:7812-7816) phenotypes in mice and the RPE65 phenotype in dogs (Acland et al. 2001, Nat. Genet. 28:92-95) when the wild type transgene is delivered to photoreceptors or the retinal pigmented epithelium (RPE) in animals with a specific mutation.

Angiogenesis is the process by which new blood vessels form. In response to specific chemical signals, capillaries sprout from existing vessels, eventually growing in size as needed by the organism. Initially, endothelial cells, which line the blood vessels, divide in a direction orthogonal to the existing vessel, forming a solid sprout. Adjacent endothelial cells then form large vacuoles and the cells rearrange so that the vacuoles orient themselves end to end and eventually merge to form the lumen of a new capillary (tube formation).

Angiogenesis is stimulated by a number of conditions, such as in response to a wound, and accompanies virtually all tissue growth in vertebrate organisms such as mammals. Angiogenesis also plays a role in certain disease states such as diabetic retinopathy and certain cancers. The growth of tumors, for example, requires blood vessel growth to provide oxygen and nutrients to the growing tumor tissue.

Angiogenesis may be arrested or inhibited by interfering with the chemical signals that stimulate the angiogenic process. For example, angiogenic endothelial cells produce proteases to digest the basal lamina that surround the blood vessels, thus clearing a path for the new capillary. Inhibition of these proteases, or their formation, can prevent new vessels from forming. Likewise, the endothelial cells proliferate in response to chemical signals. Particularly important proliferation signals include the vascular endothelial growth factor (VEGF), and the fibroblast growth factor (FGF) families of proteins. VEGF has been shown to be involved in vascularization of certain tumors. Interference with these proliferation signaling processes can also inhibit angiogenesis.

Several factors are involved in angiogenesis. Both acidic and basic fibroblast growth factor molecules are mitogens for endothelial cells and other cell types. A highly selective mitogen for vascular endothelial cells is vascular endothelial growth factor (VEGF).

In the normal adult, angiogenesis is tightly regulated, and is limited to wound healing, pregnancy and uterine cycling. Angiogenesis is turned on by specific angiogenic molecules such as basic and acidic fibroblast growth factor (FGF), VEGF, angiogenin, transforming growth factor (TGF), tumor necrosis factor-α (TNF-α) and platelet derived growth factor (PDGF). Angiogenesis can be suppressed by inhibitory molecules such as interferon-α, thrombospondin-1, angiostatin and endostatin. It is the balance of these naturally occurring stimulators and inhibitors that controls the normally quiescent capillary vasculature. When this balance is upset, as in certain disease states, capillary endothelial cells are induced to proliferate, migrate and ultimately differentiate. WO 02/067970 and WO 03/009813 disclose TRpRS fragments for use in the treatment of ocular neovascular disease by inhiting ocular neovascularisation through their angiostatic activity.

Angiogenesis plays a central role in a variety of disease including cancer and ocular neovascularization. Sustained growth and metastasis of a variety of tumors has also been shown to be dependent on the growth of new host blood vessels into the tumor in response to tumor derived angiogenic factors. Proliferation of new blood vessels in response to a variety of stimuli occurs as the dominant finding in the majority of eye disease and that blind including proliferative diabetic retinopathy, ARMD, rubeotic glaucoma, interstitial keratitis and retinopathy of prematurity. In these diseases, tissue damage can stimulate release of angiogenic factors resulting in capillary proliferation. VEGF plays a dominant role in iris neovascularization and neovascular retinopathies. While reports clearly show a correlation between intraocular VEGF levels and ischemic retinopathic ocular neovascularization, FGF likely plays a role. Basic and acidic FGF are known to be present in the normal adult retina, even though detectable levels are not consistently correlated with neovascularization. This may be largely due to the fact that FGF binds very tightly to charged components of the extracellular matrix and may not be readily available in a freely diffusible form that would be detected by standard assays of intraocular fluids.

A final common pathway in the angiogenic response involves integrin-mediated information exchange between a proliferating vascular endothelial cell and the extracellular matrix. This class of adhesion receptors, called integrins, are expressed as heterodimers having an α and β subunit on all cells. One such integrin, αᵥβ₃, is the most promiscuous member of this family and allows endothelial cells to interact with a wide variety of extracellular matrix components. Peptide and antibody antagonists of this integrin inhibit angiogenesis by selectively inducing apoptosis of the proliferating vascular endothelial cells. Two cytokine-dependent pathways of angiogenesis exist and may be defined by their dependency on distinct vascular cell integrins, αᵥβ₃ and αᵥβ₅. Specifically, basic FGF- and VEGF-induced angiogenesis depend on integrin αᵥβ₃ and αᵥβ₃, respectively, since antibody antagonists of each integrin selectively block one of these angiogenic pathways in the rabbit corneal and chick chorioallantoic membrane (CAM) models. Peptide antagonists that block all αᵥ integrins inhibit FGF- and VEGF-stimulated angiogenesis. While normal human ocular blood vessels do not display either integrin, αᵥβ₃ and αᵥβ₅ integrins are selectively displayed on blood vessels in tissues from patients with active neovascular eye disease. While only αᵥβ₃ was consistently observed in tissue from patients with ARMD, αᵥβ₃ and αᵥβ₅ both were present in tissues from patients with proliferative diabetic retinopathy. Systemically administered peptide antagonists of integrins blocked new blood vessel formation in a mouse model of retinal vasculogenesis.

Testing potential treatments for retinal neovascular diseases has been greatly facilitated by the development of models of oxygen-induced retinopathy (OIR) in several animal species, including the kitten, the beagle puppy, the rat, and the mouse. In each of these models, exposing new-born animals to hyperoxia (or to alternating hyperoxia and hypoxia) prompts regression or delay of retinal vascular development, followed by abnormal angiogenesis after their return to normal oxygen levels. These models mirror the events that occur during retinopathy of prematurity (ROP), a condition involving pathological neovascularization that can affect premature infants.

Over the last decade, the mouse model of OIR has become the most common model for studying abnormal angiogenesis associated with oxygen-induced retinopathies. The pattern of vascular abnormalities in this model differs slightly from that observed in ROP; in the mouse the central, posterior retina becomes avascular following exposure to hyperoxia while in human infants the periphery is avascular. Nonetheless, this is a well-accepted model for studying disease mechanisms and potential treatment of hypoxia-induced retinopathy, and the vascular changes are very consistent, reproducible and quantifiable. In recent years, the use of this model has been extended to the general study of ischemic vasculopathies and related anti-angiogenic interventions, and it is now used extensively in both basic and applied research environments.

The historically common method for quantifying the proliferative neovascular response in the mouse OIR model is based upon counting the number of cells associated with neovessels extending from the retina into the vitreous ("pre-inner limiting membrane (ILM) nuclei"). This is done in sagittal cross sections, usually in regions near (but not including) the optic disk. The method is very labor-intensive, time consuming, and is fraught with difficulties, including the need to differentiate cells of the abnormal vessels from those of the hyaloidal vessels in the vitreous. Because, in general, only every 30th serial section is evaluated, a large portion of the tissue is not quantified potentially introducing large sampling errors. In addition, as whole eyes are immediately sectioned, it prevents same-eye assessment of another important parameter of this model, namely the extent of vascular obliteration and rate of retinal revascularization which occurs concomitantly with abnormal neovascular tufts formation. This parameter is best assessed in retinal whole retinal mount preparations.

Accordingly, there is an ongoing need for improved methods of treating retinal neovascular diseases and for evaluating the therapeutic efficacy of such treatments in the whole retina. The methods of the present invention fulfill these needs.

### SUMMARY OF THE INVENTION

The present invention provides a use for promoting physiological revascularization of ischemic retinal tissue. The use comprises administering to a mammal suffering from retinal ischemia a therapeutically effective amount of an angiostatic fragment of tryptophanyl-tRNA synthetase (TrpRS), thereby simultaneously inhibiting pathological neovascularization of the retina while promoting beneficial physiological revascularization of damaged areas of the retina. In a preferred embodiment, the angiostatic fragment of TrpRS is the T2 fragment. Preferably, the mammal is a human patient suffering from retinal ischemia

The use of the present invention is useful for treatment of ischemic and neovascular eye diseases, including ischemic retinopathies such as diabetic retinopathy, retinopathy of prematurity, and the like.

Also disclosed is a screening method for identifying and evaluating of the therapeutic efficacy of potential therapeutic agents for treating retinal neovascular diseases. The method comprises exposing a neonatal mouse, preferably about 7 days old, to hyperoxia for a period of time sufficient to induce measurable regression of retinal vasculature, preferably about 5 days, and subsequently returning the mice to normoxia. After return to normoxia, a potential therapeutic agent is then administered to an eye of the mouse. The whole retina from the treated eye is isolated, preferably about 5 days after return to normoxia. A substantially whole retina is also isolated from a control eye from the same mouse or a mouse of the same age exposed to the same level of hyperoxia for the same amount of time. The control eye preferably has been administered a buffer or saline solution in place of the potential therapeutic agent. The blood vessels of the treated and control retinas are stained for ready identification and visualization.

Micrographic images of the substantially the entire stained retina are acquired to visualize the area of vascular obliteration caused by the hyperoxia and the area of pre-retinal neovascular tufts which form upon return to normoxia. The micrographic images are then analyzed to assess the area of vascular obliteration caused by the hyperoxic conditions and to assess the extent of pathological neovascularization that occurs upon return to normoxia. The efficacy of a potential therapeutic agent is assessed by comparing the area of vascular obliteration in treated eyes versus control eyes and by comparing the area of pre-retinal neovascular tufts in the treated eyes versus the control eyes. The extent of pathological neovascularization is evidenced by the area of the retina exhibiting pre-retinal neovascular tufts in treated eyes relative to control eyes. A decrease in the area of pre-retinal neovascular tufts indicates angiostatic activity, i.e., that the agent inhibits pathological neovascularization. Evaluation of revascularization is performed by comparing by the area of vascular obliteration in treated relative to the control eyes. A reduction in the area of vascular obliteration in treated eyes versus controls indicates that the therapeutic agent induces beneficial physiological revascularization of damaged retinas.

### BRIEF DESCRIPTION OF THE DRAWINGS

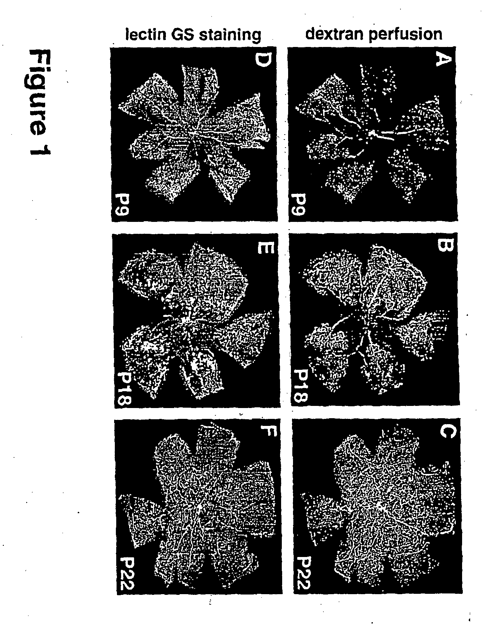

Figure 1 depicts photomicrographic montages of stained, whole retinas from neonatal mice, illustrating the normal development of vasculature in the mouse eye from postnatal age 9-days (P9) to age 22-days (P22). Panels A, B and C illustrate vasculature at P9, P 18, and P22, respectively, visualized by staining with FITC-dextran perfusion. Panels D, E and F illustrate vasculature at P9, P18, and P22, respectively, visualized by staining with lectin GS.

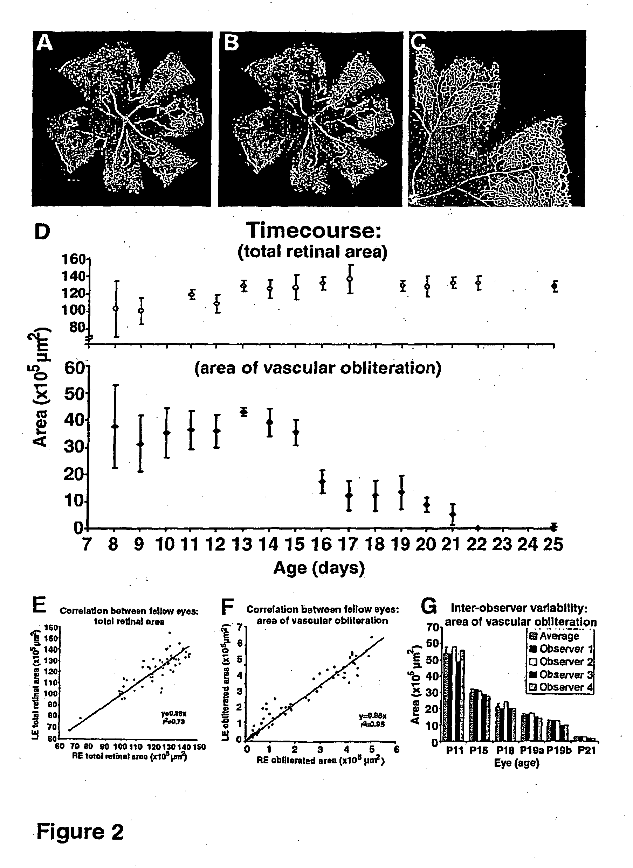

Figure 2 depicts photomicrographic montages and graphs illustrating the development of retinal vasculature in neonatal mice exposed to hyperoxia during the period of P7 to P12. Panels A, B, and C are photomicrographs showing areas of vascular obliteration. Panel D (upper graph) is a graph of retinal area versus age. Panel D (lower graph) is a graph of area of vascular obliteration versus age. Panel E is a scatter plot showing the correlation of left eye retinal area with right eye retinal area. Panel F is a scatter plot showing the correlation of left eye vascular obliterated area with right eye vascular obliterated area. Panel G is a bar graph showing the low observer to observer variability of measured area of vascular obliteration for 6 different retinas isolated at different ages measured by 4 different observers according to the screening method of the invention.

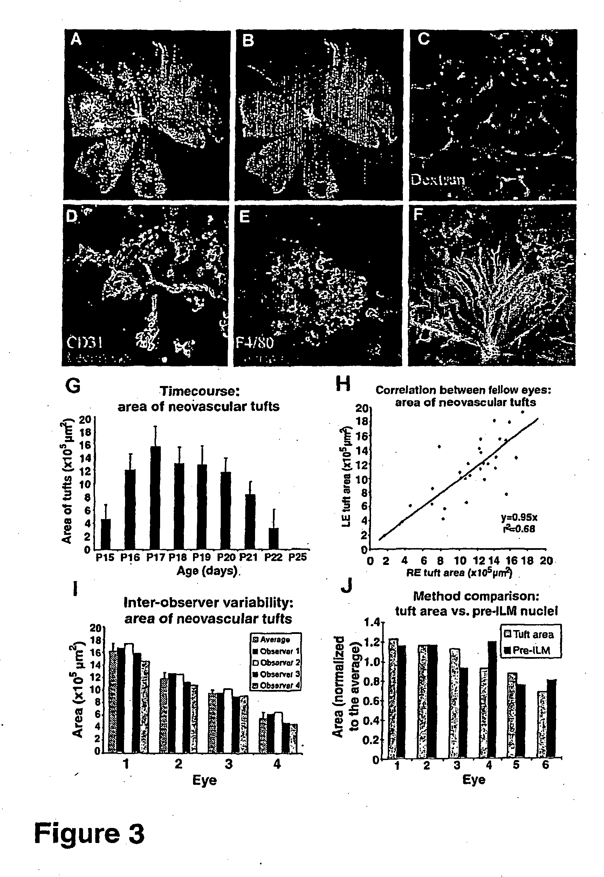

Figure 3 depicts photomicrographs and graphs illustrating the development of pre-retinal neovascular tufts in neonatal mice exposed to hyperoxia during the period of P7 to P12. Panels A-F are photomicrographs in which Panel A is imaged to show pre-retinal neovascular tufts (bright portions). Panel B shows the image of Panel A with the vascular tufts in red. Panel C shows that areas of neovascular tufts are only partially perfused - tufts connected to the underlying vasculature are shown in green. Panel D shows that the isolectin GS-stained cells are predominately CD31 positive (green), indicating vascular lineage. Panel E shows that some cells of macrophage lineage (green) are present among the CD31 positive neovascular tufts. Panel G is a graph showing the area of neovascular tufts as a function of age. Panel H shows a correlation of neovascular tuft area of the right eye versus the left eye. Panel I is a bar graph illustrating low inter-observer variability in determination of neovascular tuft area according to the screening method of the invention. Panel J shows the correlation between tuft area as measured by the screening method of the invention versus the labor intensive prior art cross-section method of measuring pre-ILM nuclei.

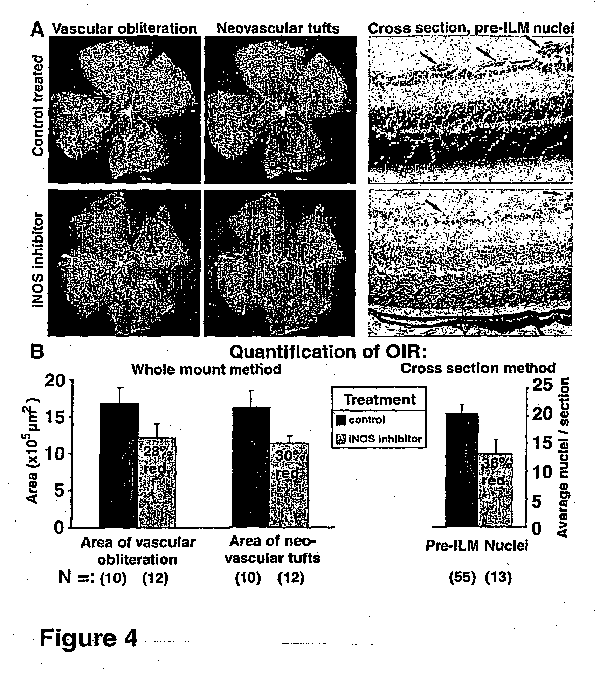

Figure 4 depicts photomicrographs and graphs illustrating quantification of vascular obliteration and pre-retinal neovascular tuft areas in neonatal mice exposed to hyperoxia during the period of P7 to P12, according to the screening method of the invention. Panel A: (upper right image) shows the area of vascular obliteration of a control mouse eye exposed to hyperoxia according to the screening method of the invention; (upper middle image) shows area of neovascular tufts (red) for the control mouse eye; (upper left image) is a cross section of the control retina showing pre-ILM nuclei (arrows). Panel B: (lower right image) shows the area of vascular obliteration of a mouse eye exposed to hyperoxia and treated with an iNOS inhibitor according to the screening method of the invention; (lower middle image) shows area of neovascular tufts (red) for the treated mouse eye; (lower left image) is a cross section of the treated retina showing pre-ILM nuclei (arrows). Panel B: (left graph) compares area of vascular obliteration for the control eye versus the iNOS treated eye; (middle graph) compares area of neovascular tufts for the control eye versus the iNOS treated eye; (right graph) compares the number of pre-ILM nucei for the control eye versus the iNOS treated eye.

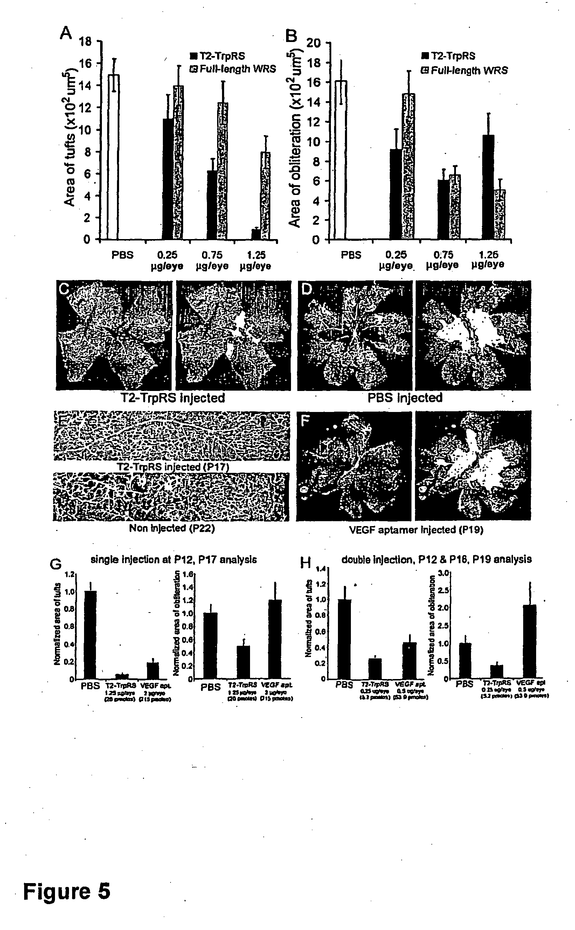

Figure 5 depicts photomicrographs and graphs illustrating quantification of the ameliorating effects of an angiostatic fragment of TrpRS on vascular obliteration and neonatal tuft formation in neonatal mice exposed to hyperoxia during the period of P7 to P 12, according to the screening method of the invention. Panel A is a graph comparing neovascular tuft area for eyes treated at various doses with T2-TrpRS compared to eyes treated with similar doses of full length TrpRS in the screening method of the invention. Panel B is a graph comparing vascular obliteration area for eyes treated with various doses of T2-TrpRS compared to eyes treated with similar doses of full length TrpRS in the screening method of the invention. Panels C and D illustrate the reduced area of vascular obliteration for T2-TrpRS treated eyes (C) versus control eyes injected with PBS buffer (D). Panel E illustrates the accelerated revascularization induced by T2-TrpRS (top image) versus control eye (bottom image). Panel F illustrates that VEGF aptamer causes a delay in revascularization of vascular obliterated areas. Panels G and H are graphs comparing the activity of T2-TrpRS and VEGF aptamer in the screening method of the present invention.

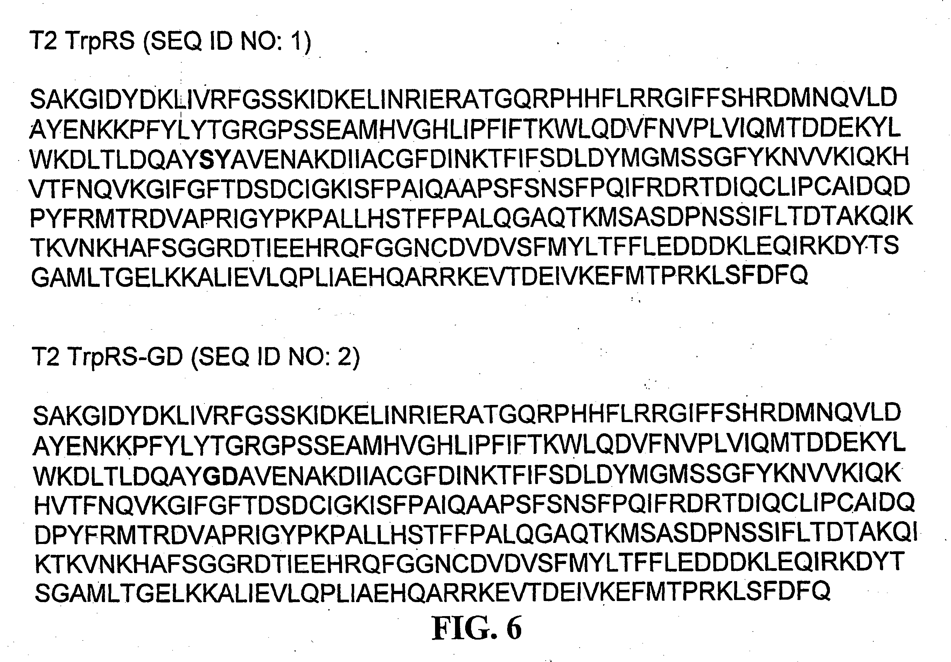

Figure 6 Illustrates the amino acid residue sequence of T2-TrpRS (SEQ ID NO: 1) and mutant T2-TrpRS-GD (SEQ ID NO: 2).

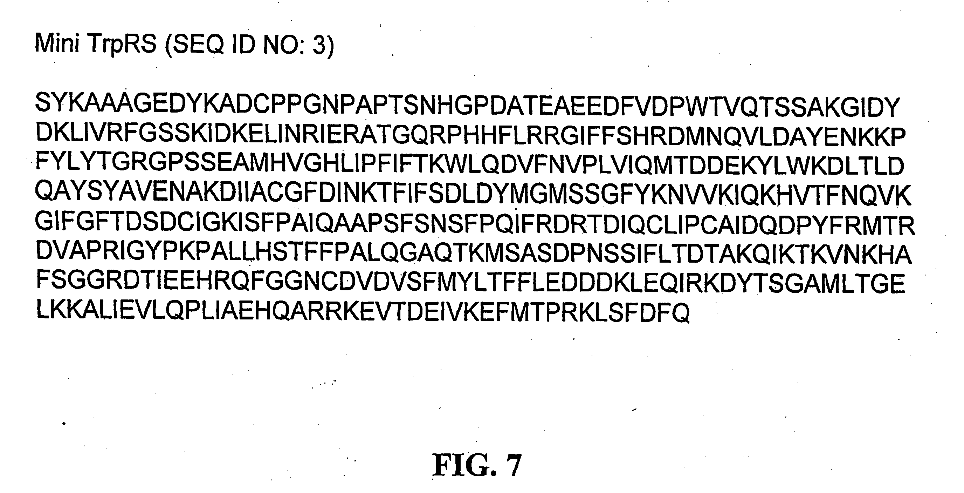

Figure 7 Illustrates the amino acid residue sequence of mini-TrpRS (SEQ ID NO: 3).

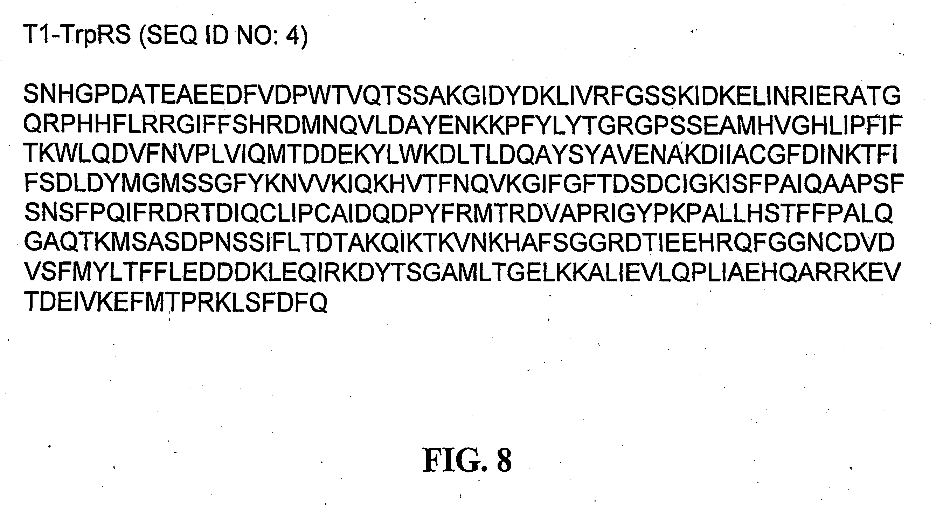

Figure 8 Illustrates the amino acid residue sequence of T1-TrpRS (SEQ ID NO: 4).

### DETAILED DESCRIPTION OF PREFERRED EMBODIMENTS

A method of promoting beneficial physiological revascularization of ischemic retinal tissue comprises administering to a mammal suffering from retinal ischemia a therapeutically effective amount of an angiostatic fragment of tryptophanyl-tRNA synthetase (TrpRS), thereby simultaneously inhibiting pathological neovascularization while promoting physiological revascularization of damaged areas of the retina. Preferably, the TrpRS fragment is administered by intravitreal injection into the eye.

Preferred angiostatic fragments of TrpRS include the T2 fragment (T2-TrpRS; SEQ ID NO: 1, FIG. 6), a mutant of T2-TrpRS (T2-TrpRS-GD; SEQ ID NO: 2, FIG. 6), a truncated TrpRS known as mini-TrpRS (SEQ ID NO: 3, FIG. 7) and T1-TrpRS (SEQ ID NO: 4, FIG. 8). The amino acid residue sequence of T2-TrpRS-GD (SEQ ID NO: 2), differs from SEQ ID NO: 1 by two amino acid residue substitutions (i.e., S121G and Y122D). More preferably, the angiostatic fragment of TrpRS is the T2 fragment.

Also disclosed is a screening method for evaluating of the therapeutic efficacy of putative therapeutic agents for treating retinal neovascular diseases. The method utilizes a mouse model of oxygen-induced retinopathy to simulate a retinopathic disease state in the eye of a mouse. The method comprises exposing neonatal mice (preferably about 7 days old) to hyperoxia (e.g., a 75% oxygen atmosphere) for a period of time sufficient to induce measurable regression of retinal vasculature, preferably about 5 days, and subsequently returning the mice to normoxia (i.e., normal air). After return to normoxia, an eye of each mouse is then administered a potential therapeutic agent. Preferably, one eye of an individual mouse is administered the therapeutic agent, while the other eye is administered a placebo, such as saline or a buffer solution, as a control. Alternatively, some mice can be utilized as controls while others are treated with the potential therapeutic agent. Whole retinas from the treated and control mice are isolated, preferably about 5 days after return to normoxia, and the blood vessels thereof are stained for ready identification.

Micrographic images of substantially the entire stained retinas are acquired and analyzed to assess the area of vascular obliteration caused by the hyperoxic conditions and to assess the extent of pathological neovascularization that occurs upon return to normoxia. The efficacy of a potential therapeutic agent is assessed by comparing the area of vascular obliteration in treated eyes versus control eyes and/or by comparing the area of pre-retinal neovascular tufts in the treated eyes versus the control eyes.

A preferred screening method for identifying and evaluating the therapeutic efficacy of potential therapeutic agents for treating retinal neovascular diseases comprises exposing a neonatal mouse to hyperoxia for a period of time sufficient to induce measurable regression of retinal vasculature and returning the mice to normoxia. Next, a solution of putative therapeutic agent is administered to an eye of the mouse. After a period of up to about 5 days, the mouse is euthanized and substantially the entire retina is removed from the eye to which the putative therapeutic agent was administered. The vasculature of the retina is stained, and substantially the entire stained retina is micrographically imaged to visualize the vasculature of the retina. At least one of (a) the area of vascular obliteration observable in the stained retina, and (b) the area of pre-retinal neovascular tufts in the stained retina, is determined from the image. Subsequently, a comparison is made of at least one of (i) the area of vascular obliteration observable in the stained retina compared to the area of vascular obliteration observable in a stained retina from a control eye of a mouse exposed to the same conditions of hyperoxia but not administered the putative therapeutic agent and (b) the area of pre-retinal neovascular tufts in the stained retina compared to the area of pre-retinal vascular tufts in a stained retina from a control eye of a mouse exposed to the same conditions of hyperoxia but not administered the putative therapeutic agent.

A preferred screening method for identifying and evaluating the therapeutic efficacy of potential therapeutic agents for treating retinal neovascular diseases comprises:
exposing a 7-day old neonatal mouse to an atmosphere containing about 75% oxygen for about 5 days (hyperoxic conditions);
returning the mice to an atmosphere of normal air (normoxia);
administering a potential therapeutic agent to an eye of the mouse after return to normal air;
euthanizing the mouse and removing substantially the entire retina from the eye to which the putative therapeutic agent was administered;
staining the vasculature of the retina of the eye to which the putative therapeutic agent was administered;
preparing at least one micrographic image of substantially the entire stained retina to visualize the vasculature thereof;
determining the area of vascular obliteration observable in the stained retina and the area of pre-retinal neovascular tufts in the stained retina from the at least one micrographic image;
comparing at the area of vascular obliteration observable in the stained retina to the area of vascular obliteration observable in a stained retina from a control eye of a mouse exposed to the same hyperoxic conditions, the control eye not having been administered the putative therapeutic agent; and
comparing the area of pre-retinal neovascular tufts in the stained retina to the area of pre-retinal vascular tufts observable in a stained retina from a control eye of a mouse exposed to the same hyperoxic conditions, the control eye not having been administered the putative therapeutic agent.

The extent of pathological neovascularization is evidenced the area of the retina exhibiting pre-retinal neovascular tufts in treated eyes relative to control eyes visible in the micrographic image. A decrease in the area of pre-retinal neovascular tufts indicates angiostatic activity, i.e., that the agent inhibits pathological neovascularization. Evaluation of revascularization is performed by comparing the area of vascular obliteration in retinas from treated eyes versus control eyes. A reduction in the area of vascular obliteration in retinas from treated eyes versus controls indicates that the therapeutic agent induces beneficial physiological revascularization of damaged retinas.

Therapeutic compositions comprising an angiostatic TrpRS fragment can include pharmacologically appropriate, pharmaceutically acceptable carriers, excipients and vehicles. In general, these carriers include aqueous or alcoholic/aqueous solutions, emulsions or suspensions, including saline and buffered media such as phosphate buffered saline (PBS). Parenteral vehicles can include sodium chloride solution, Ringer's dextrose, dextrose and sodium chloride, lactated Ringer's or fixed oils. In addition, intravenous vehicles can include fluid and nutrient replenishers, and electrolyte replenishers, such as those based on Ringer's dextrose. Excipients such as preservatives and other additives can also be present, such as, for example, antimicrobials, antioxidants, chelating agents, and inert gases. Suitable formulation aids, carriers, other excipients, and methods of formulating pharmaceutical compositions are disclosed in Remington's Pharmaceutical Sciences, 14th Ed., Mack Publishing Co., 1970, particularly Part VIII, "Pharmaceutical Preparations and Their Manufacture", pages 1461 - 1762, the relevant disclosures of which are incorporated herein by reference.

The therapeutic compositions can be packaged in suitably sterilized bottles or vials, either in multi-dose or in unit dosage forms. The containers are preferably hermetically sealed after being filled with a composition of the invention. Preferably, the compositions are packaged in a container having a label affixed thereto, which label identifies the drugs present in the composition, and bears a notice in a form prescribed by a government agency such as the United States Food and Drug Administration, reflecting approval of the composition under appropriate laws, dosage information, and the like. The label preferably contains information about the composition that is useful to a health care professional administering the composition to a patient. The package also preferably contains printed informational materials relating to the administration of the composition, instructions, indications, and any necessary required warnings.

### Oxygen induced retinopathy (OIR) model.

All animal work adhered to strict protocol guidelines for the humane care and use of animals in research. OIR was induced according to the protocol described by Smith et al. Invest. Opthalmol. Vis. Sci., 1994; 35:101-111. Seven-day old mouse pups and their mothers were transferred from room air to a hyperoxic environment of about 75% oxygen for 5 days, and afterwards returned to room air (normoxia). Exposure to hyperoxic conditions was performed by putting the animal cages within a modified human infant incubator. Oxygen was circulated through the incubator (using an animal anesthesia machine to control flow) and levels were monitored and maintained at 74 - 75% using an FDA-approved oxygen analyzer (AX-300, Teledyne Analytical Instruments, CA, USA). During the 5 days of hyperoxia, the animals were examined at least three times daily. Unlimited food and water were available to the mothers throughout the experiment. After 5 days, i.e., at postnatal Day 12 (P12), cages were returned to room air.

### Retinal whole mount preparation and immunohistohemistry

After the mice were euthanized, enucleated eyes were fixed in 4% paraformaldehyde in PBS for about 10 minutes, and the retinas were dissected by making a circular cut behind the limbus, approximately at the iris root, and then removing the anterior part of the eye. The lens and vitreous were dissected out, and the retina was separated from the scleral and choroidal layers leaving the retina in an eye-cup configuration. Radial relaxing-incisions were made in the retina to allow it to flatten and any remnants of the vitreous or hyaloid were carefully removed from the flattened retina.

For FITC-dextran angiography, animals were anaesthetized by intraperitoneal injections of Ketamine HCl (Ketalar, Parke Davis, UK, 100mg/kg) in combination with the relaxing agent Xylazine (2mg/kg). Approximately 300µl of high molecular weight (2 x 106 Da) FITC-Dextran (concentration 50mg/ml, Sigma, St. Louis, MO) were injected into the left ventricle and allowed to circulate for approximately two minutes before euthanizing the animal and enucleating the eyes. For isolectin G4 staining, retinas were post-fixed in 4% paraformaldehyde for about 45 minutes, and incubated at room temperature overnight with isolectin GS-IB4 from *Griffonia simplicifolia* (isolectin GS) that was conjugated to Alexa Fluor 59 4 (Molecular Probes, 1:150 dilution in phosphate buffered saline (PBS)) to afford "isolectin GS-594". Following 4-5 washes with PBS over a 2 hour span, retinas were mounted as whole-mount preparations on slides with SLOW FADE (Molecular Probes) for subsequent imaging.

Since isolectin GS will stain not only vascular endothelial cells but also activated murine macrophages, polyclonal antibodies specific for the endothelial marker CD31 (BD Biosciences- Pharmingen, 1:100 dilution in 10% fetal bovine serum (FBS) /10% NGS buffer) and antibodies against the macrophage marker F4/80 (1:150) were used in a subset of retinas to further characterize the isolectin GS-positive cells. Retinas were blocked with 20%FBS, 20% NGS in PBS for 1 hour at room temperature and incubated with the primary antibody and isolectin GS-594 overnight. Following washes with PBS, Alexa 488-conjugated secondary antibodies (Invitrogen Corporation) were applied for 2 hours, washed, and retinas mounted as described above.

### Imaging and Quantification

Micrographic images were taken using a 4x objective lens with a detector resolution of 512 x 512 pixels. Extra care was taken to focus just above the inner limiting membrane of the retina using confocal microscopy (BioRad) so that the pre-laminar neovascular tufts became more prominent than the underlying superficial vascular plexus making subsequent quantification of the neovascular tuft areas easier. In most cases, four overlapping images (each representing one retinal quadrant) were acquired from each retina. Using Adobe PHOTOSHOP® 6.0, montages were then created using retinal landmarks such as the optic disc and major vessels to align the images. These montages allowed visualization and quantification of the entire retina. In a few cases where both the obliterated areas and all areas of neovascularization were located toward the center of the retina, single 4x images centered on the optic disk were acquired. While the peripheral retina is not included in its entirety in such images, the relevant OIR-induced changes can be fully seen in these cases. Each individual image was converted to a 2 x 2 inch size with 300 pixels per inch resolution prior to creating the montage and quantifying the obliteration and neovascular tuft areas.

After the images were generated and the retinal montages were created, vascular obliteration and abnormal neovascular formations were quantified by individuals in a blind protocol to prevent bias and the actual retinas were visualized under the microscope whenever necessary to help determine the actual areas of obliteration and neovascular tufts in the images. To measure areas of vascular obliteration, the Adobe PHOTOSHOP® freehand selection tool was used. The borders of the avascular areas were traced and the total area of obliteration in pixels was computed. Measurement of the areas of neovascular tufts was also performed in the whole retinal mount configuration and was based upon the higher intensity of isolectin staining in these regions, their characteristic appearance, and images created by focusing above the retina's inner limiting membrane. The neovascular tufts were specifically selected by hand using the magic wand tool in Adobe PHOTOSHOP® software. The total area in pixels was then determined. Once areas were measured in pixels, a conversion factor was applied, which for our parameters was calculated to be 27.8 µm² per pixel, and absolute areas (in µm²) were derived. This conversion factor was computed based on image acquisition (4x magnification lens) and montage assembly parameters such size (2 x 2 inches) and resolution (300 pixels per inch) upon importing and creating the montages in Adobe PHOTOSHOP® software.

### Retinal cross sections and pre-ILM nuclei counting

In a subset of six eyes, following quantification in the whole mount configuration, the retinas were removed from the slides and processed for cross sectioning and pre-ILM nuclei counting. Following additional fixation in 4% PFA, retinas were embedded in paraffin and serially sectioned in their entirety. Standard H&E staining was performed, and all nuclei present on the vitreal side of the ILM were counted. The average number of nuclei per section was computed. In an additional set of eyes treated with an iNOS inhibitor (described below), whole eyes were embedded in paraffin, cross sectioned, stained with H&E, and a sample of cross sections was counted as previously described by Smith *et al.* The optic nerve was identified, and 2-4 sections on each side of it, 30 to 90 µm apart, were counted for neovascularization.

### Injection of angiostatic compounds

Intravitreal injections were made at P12 (upon return to normoxia) for the single injection treatments, or at P13 and P16 for the double injection treatments. In each case, 0.5 µL volumes of the appropriate solution (PBS control, PBS solutions containing individual therapeutic agent, or PBS solutions containing combination therapies) were injected using a Hamilton syringe fitted with a 33-gauge needle. Injections were made between the equator and the corneal limbus. During injection, the location of the needle was visualized through a dissecting microscope to ensure that it was in the vitreous cavity, and thus, minimize the possibility of retinal or lens damage. After the injection, the eyelids were repositioned to close the fissure.

### Results

The normal process of post-natal retinal vascular development in the mouse is well characterized, and includes centripetal growth of superficial vessels emerging from the optic disc at birth and reaching the far periphery of the retina approximately 9-11 days later. Near the end of the first week after birth, short vessels begin to dive down from this superficial network to form the deep vascular network, which usually is complete by about P11-12. Then, a final, intermediate layer of vessels forms (IL). The process is accompanied by waves of remodeling and pruning, and is largely completed by about P21-23. In neonatal C57B16/J mice, exposure to high levels of oxygen significantly alters this orderly sequence of events. Following exposure to 75% oxygen between P7 and P12, large areas of the vascular network regress in the center of the retina (vascular obliteration), leaving only the major vessels and practically no capillary network. This can be seen in both dextran-perfused (Figure 1, Panel A) and isolectin-stained (Figure 1, Panel D) whole mount preparations of a P9 eye. Isolectin-positive macrophages were also present within the area of vascular obliteration (Figure 1, Panel D and also Figure 2, Panel C). The peripheral retina still shows evidence of a vascular network comprised mainly of superficial vessels (Figure 2, Panel C). Upon return to normoxia at P 12, a relative state of ischemia in the poorly vascularized retina prompts re-growth of the superficial vessels from the periphery inwards accompanied by vascular sprouting within the retina to form the deep vascular layer and also abnormal sprouting at the interface between retina and vitreous (pre-retinal vascular tufts). By about P18, the areas of vascular obliteration are smaller, extending around the optic disc and along the main retinal arteries (Figure 1, Panel B and E), and the pre-retinal tufts are evident as brightly stained, lectin-positive regions (Figure 1, Panel E, Figure 3, Panel A). It is important to note that following perfusion of the retinal vessels with a fluorescent dye (in this case Dextran FITC), the areas of vascular obliteration are clearly delineated but the tufts cannot be easily identified (compare Figure 1, Panel B to Panel E). By about P22-23, the retina is usually largely revascularized (Figure 1, Panel C and Panel F) and the tufts are mostly resolved although a few more days may pass before it assumes a fully normal appearance.

### Example 1. Quantifying Areas of Vascular Obliteration and Rate of Retinal Revascularization.

In order to quantify vascular obliteration and the rate of retinal revascularization, dextran-injected or isolectin stained retinal whole mount preparations were imaged using fluorescent microscopy at low magnification. Four overlapping images were used to construct a montage of the retina (Figure 2, Panel A), and the area of obliteration (yellow) as well as the total retinal area (blue line) were measured using readily available computerized image processing programs (Figure 2, Panel B). Since the border between the vascular and avascular retina is usually well defined in these preparations, inter-observer variability was very low (Figure 2, Panel G). Care was taken not to include in the analysis areas in which a dissection or mounting artifact was present. Changes in total retinal area over time are shown in the upper panel of Figure 2, Panel D. Retinal growth apparently continues during hyperoxia and total retinal area grows by approximately 20% between P8 and P13. Later, the rate of change decreases. The time course of vessel obliteration and revascularization (Figure 2, Panel D, lower graph) shows that obliteration occurs quite rapidly following exposure to hyperoxia at P7 and occupies approximately 30-35% of the total retinal area between P8-P12. Revascularization after return to normoxia occurs at a slower rate with extensive neovascularization beginning around P 16, four days after return to normoxia. The process is quite symmetrical, as demonstrated by the high degree of correlation between fellow eyes (i.e., eyes of the same animal) in terms of total retinal area (Figure 2, Panel E) and especially in extent of vascular obliteration (Figure 2, Panel F). Preferably, fellow eye controls are used in practicing the methods of the present invention.

### Example 2. Quantifying Extent of Abnormal Neovascularization.

Between P15 and P22, and particularly from P17-P19, abnormal vascular growth occurs at the interface between the retina and the vitreous in the mouse OIR model. These pre-retinal vascular tufts are especially obvious at the border between the vascularized peripheral and obliterated central regions of the retina. These misdirected vascular elements have classically been quantified in H&E stained, paraffin-embedded cross sections by counting nuclei of cells protruding to the vitreal side of the inner limiting membrane (ILM). However, these areas can also be seen quite clearly in isolectin-stained whole mount images, as collections of cells overlying the superficial vascular network (Figure 1, Panel E, Figure 3, Panel A). Using either an intensity threshold tool (available in a number of commercial image analysis programs), or Adobe PHOTOSHOP® software to select the regions of pre-retinal tuft formation by hand, the area of these strongly-staining isolectin-positive regions can be readily measured (Figure 3, Panel B, neovascular areas marked in red). Confocal microscopy assists in clarifying the location of these cells, as well as their relation to the underlying retinal blood vessels (Figure 3, Panel C). Note that these areas of proliferation are only partially perfused, as seen by the green spots of intravenously-injected dextran-FITC within them (Figure 3, Panel C). This limited level of perfusion precludes using dextran-FITC angiography to adequately visualize and quantify the tufts. Cell-specific markers show that the majority of isolectin-positive cells within the tufts express the endothelial marker CD31, confirming their vascular identity (Figure 3, Panel D). Interestingly, cells of macrophage lineage are also present in the clumps as shown by F4/80 immunostaining (Figure 3, Panel E).

The extent and time course of abnormal vascularization is shown in Figure 3, Panel G. Between P16-P20, approximately 12-16% of the total retinal area is covered by neovascular tufts, peaking at P17. It is important to note that continuous and rapid remodeling of these tufts occurs between P15-P22. In the wake of the retinal revascularization, which is concomitantly proceeding with the remodeling, the remodeled areas also resolve. The relatively large, protruding groups of cells seen earlier (Figure 3, Panel A-E) are pruned down to rows of isolectin-positive cells, which generally follow the retinal vessels (Figure 3, Panel F) and ultimately disappear between P22-P25 (Figure 1, Panel F).

While the correlation between fellow eyes, in terms of tuft formation, is not as good as that seen for vascular obliteration, it is none-the-less a largely symmetrical process (Figure 3, Panel H). Inter-observer variability was again low regardless of the timepoint studied and the extent of apparent tuft formation (Figure 3, Panel I), attesting to the relative consistency and clarity of the quantification method of the present invention. Finally, in order to examine whether the neovascular areas measured in whole mounts correlate with pre-ILM (i.e., pre-retinal) nuclei counting in cross sections, a number of previously-quantified whole mounted retinas were paraffin embedded, cross sectioned, and the pre-ILM nuclei were counted in all sections. Figure 2, Panel J shows the ratios of tuft area and of nuclei counts in each of 6 different eyes, normalized to the average of each parameter. It is evident that these two methods of quantification are generally in agreement (i.e., an eye with a larger tuft area tends to also have a higher nuclei count and vice versa). The quantification protocol of the present method has advantages over the cross-sectional counting method, however, in that the whole retinal is evaluated at once.

### Example 3. The anti-angiogenic effect of an iNOS inhibitor.

The utility of the quantification technique of the present invention was further assessed and compared with the prior art method of pre-ILM nuclei counting by examining the effect of a novel anti-angiogenic agent, an inhibitor of iNOS, on retinal revascularization and angiostasis. The inhibitor (or control saline) was delivered between P12-P17 by once-daily intra-peritoneal injections mouse eyes, and at P17 the eyes were enucleated and processed for quantification by one of the two techniques. Figure 4, Panel A shows whole mount preparations and cross section of retinas from P17 control mice (top row) and iNOS inhibitor-treated animals (Bottom row). Areas of vascular obliteration are delineated in the left panels (blue lines), areas of neovascular tufts are marked on the same whole mounts in the center panels (red), and examples of pre-ILM nuclei are shown in the cross sections on the right (arrows). Injection of the iNOS inhibitor reduced area of vascular obliteration by about 28% as compared to controls (Figure 4, Panel B, left bars). The neovascular tuft area, as quantified from whole mounts, was reduced by about 30% (center bars), which is similar to the magnitude of effect observed when pre-ILM nuclei were counted in cross sections of (i.e., about 36%, graph on lower right).

### Example 4. T2-TrpRS potently inhibits neovascular tuft formation in the OIR model.

T2-TrpRS is a proteolytic fragment of tryptophan tRNA synthetase (TrpRS) that has demonstrated potent angiostatic potential in several models of angiogenesis, including the mouse Matrigel model and the neonatal mouse retinal angiogenesis model. The method of the present invention was used to test the activity of T2-TrpRS on pathological neovascularization in the mouse OIR model. Using the present method of quantification, the effects of T2-TrpRS injection on revascularization of the superficial retinal plexus following oxygen-induced vascular obliteration was also assessed. When injected intravitreally at P12, just as the mice were returned from hyperoxia (75% O₂) to normoxia, T2-TrpRS markedly reduced the area of neovascular tuft formation at P 17 in a dose-dependent matter (Figure 5). The full-length tryptophan tRNA synthetase (FL-TrpRS) also demonstrated some activity at the highest dose, but significantly lower levels of inhibition were observed when compared to equivalent injections of T2-TrpRS. As the FL-TrpRS was previously found to have no angiostatic activity in other models of angiogenesis, it is possible that the observed activity results from natural cleavage of the FL-TrpRS to a product similar to T2-TrpRS after in vivo injection.

The obliterated areas were also quantified in control- and T2-TrpRS-treated retinas to analyze the effects on physiological revascularization of the retina following oxygen-induced obliteration (Figure 5, Panel B). Although T2-TrpRS potently inhibited neovascular tuft formation, revascularization of the normal superficial and deep retinal vascular plexuses was not inhibited. In fact, T2-TrpRS injected retinas consistently showed significantly *enhanced* revascularization, even at lower doses. In T2-TrpRS injected retinas, the obliterated areas at P17 were consistently reduced by around 40% compared to the obliterated areas in P17 PBS control-injected retinas (Figure 5, Panel B). The obliterated areas at the time of injection (P12) were about equal (data not shown). Thus, the significant and unexpected reduction in the area of obliteration at P 17 suggests a novel activity for T2-TrpRS in promoting physiological revascularization while simultaneously inhibiting pathological neovascularization. After injection of 1.25 mg/eye T2-TrpRS, the vascular morphology of the retina looked nearly normal, especially compared to normal or control-injected OIR retinas (Figure 5, Panel C-E). Even at P22, when the neovascular tufts have naturally regressed and the retina is healing, the neovessels in control OIR retinas look severely abnormal compared to the P17 T2-TrpRS injected retinas (Figure 5, Panel E). Retinas injected with FL-TrpRS also demonstrated significant enhancement of the revascularization process but only at the higher doses.

The activity of T2-TrpRS in the OIR model was directly compared to that of a VEGF aptamer that has proven properties as an antagonist of VEGF₁₆₅ activity, and has demonstrated potent inhibition of OIR-induced neovascular tuft formation. Both angiostatic molecules were potent inhibitors of neovascular tuft formation. Eyes injected with 1.25 mg/eye T2-TrpRS exhibited a slight, but significant reduction in the neovascular tuft areas compared to the VEGF aptamer treated retinas. Thus, even after injection of molar concentrations 10-fold below the aptamer, T2-TrpRS exhibited highly competitive inhibitory activity on neovascular tuft formation in the OIR model. Unlike T2-TrpRS, the VEGF aptamer *did not* enhance the revascularization of the superficial plexus. In fact, although the VEGF aptamer did not prevent physiological revascularization from occurring, the revascularization was consistently delayed compared to control vehicle (PBS) injected retinas (Figure 5, Panel F-G). This delay in the revascularization process for VEGF aptamer-treated eyes was even more obvious at P 19 after two separate injections of lower doses (Figure 5, Panel H).

### Discussion.

The presently accepted method to quantify abnormal neovascularization in the OIR model is based upon counting of pre-laminar nuclei in H&E-stained, paraffin-embedded histological cross sections of the eye. This method is very labor-intensive, time consuming, is quite variable and observer-dependant, and it also prevents accurate quantification of the extent of vascular obliteration in the same eye since retinal whole mounts are not generated. While analysis of avascular areas have been previously reported using whole mount analysis, to our knowledge, the method of the present invention is the first screening method that can be used to quantify the absolute areas of neovascular tuft formation and obliteration in a same-eye analysis. The present method uses routine tissue excision and staining methods and readily available computer programs, making it easily masterable with low inter-observer variability. In addition, this novel method also has the advantages of reducing sampling error since whole retinas are quantified rather than a limited number of sections. Differentiating vitreal nucleii associated with the hyaloid system from those associated with neovascular tufts can also be difficult in prior art cross section methods, particularly in the OIR model in which persistence of the hyaloid system occurs. In the present method, the hyaloidal vessels are generally removed during dissection and any remnants that remain, are easily identified and omitted from the analysis.

The present screening method was used to assess the angiostatic activity of T2-TrpRS in the OIR model of pathological angiogenesis. A strong, dose-dependent angiostatic effect of T2-TrpRS on pathological neovascularization in the OIR model was observed. Injection of about 1.25 µg/eye T2-TrpRS led to nearly complete inhibition of neovascular tuft formation. These observed effects were comparable, if not superior to, the of a VEGF aptamer, another potent inhibitor of pathological neovascularization in the OIR model. The longer-term effects on OIR-associated neovascularization were assessed by analyzing retinal neovascularization on P19, after two injections of lower dose inhibitors. T2-TrpRS inhibited pathological neovascularization *and* reduced tuft formation by over 75% compared to a 55% reduction after treatment with the VEGF aptamer (0.5 mg/eye/injection, 53.9 picomoles).

Surprisingly, T2-TrpRS also enhanced the beneficial physiological revascularization process following oxygen induced retinopathy. Although the areas of obliteration were equivalent at the time of injection, the obliterated areas in T2-TrpRS treated retinas were consistently reduced by half compared to control retinas. Accordingly, treatment with T2-TrpRS leads to surprisingly faster and more complete retinal healing, and a resulting vasculature that anatomically more closely resembles a normal retinal vasculature.

## Claims

1. Use of an angiostatic fragment of tryptophanyl-tRNA synthetase (TrpRS) for the preparation of a pharmaceutical composition for promoting beneficial physiological revascularization of ischemic retinal tissue wherein the pharmaceutical composition is for administering to a mammal suffering from retinal ischemia a therapeutically effective amount of an angiostatic fragment of TrpRS sufficient to inhibit pathological neovascularization and to promote physiological revascularization of ischemic areas of the retina.

2. The use of claim 1 wherein the angiostatic fragment of TrpRS is T2-TrpRS (SEQ ID NO: 1).

3. The use of claim 1 wherein the angiostatic fragment of TrpRS is T2-TrpRS-GD (SEQ ID NO: 2).

4. The use of claim 1 wherein the angiostatic fragment of TrpRS is mini-TrpRS (SEQ ID NO: 3).

5. The use of claim 1 wherein the angiostatic fragment of TrpRS is T1-TrpRS (SEQ ID NO: 4).

## Patentansprüche

1. Verwendung eines angiostatischen Fragments von Tryptophanyl-tRNA-Synthetase (TrpRS) zur Herstellung einer pharmazeutischen Zusammensetzung für eine Förderung von günstiger physiologischer Revaskularisierung von ischämischem Retinagewebe, wobei die pharmazeutische Zusammensetzung einer Verabreichung an ein Säugetier, das an retinaler Ischämie leidet, einer therapeutisch wirksamen Menge eines angiostatischen Fragments von TrpRS dient, die ausreichend ist, um pathologische Neovaskularisierung zu hemmen und physiologische Revaskularisierung- ischämischer Bereiche der Retina zu fördern.

2. Verwendung nach Anspruch 1, wobei das angiostatische Fragment von TrpRS T2-TrpRS (SEQ ID NO: 1) ist.

3. Verwendung nach Anspruch 1, wobei das angiostatische Fragment von TrpRS T2-TrpRS-GD (SEQ ID NO: 2) ist.

4. Verwendung nach Anspruch 1, wobei das angiostatische Fragment von TrpRS mini-TrpRS (SEQ ID NO: 3) ist.

5. Verwendung nach Anspruch 1, wobei das angiostatische Fragment von TrpRS T1-TrpRS (SEQ ID NO: 4) ist.

## Revendications

1. Utilisation d'un fragment angiostatique de tryptophanyl-ARNt synthétase (TrpRS) pour la préparation d'une composition pharmaceutique pour favoriser la revascularisation physiologique bénéfique du tissu rétinien ischémique, dans laquelle la composition pharmaceutique est destinée à l'administration à un mammifère souffrant d'une ischémie rétinienne d'une quantité thérapeutiquement efficace d'un fragment angiostatique de TrpRS suffisante pour inhiber la néovascularisation pathologique et pour favoriser la revascularisation physiologique de zones ischémiques de la rétine.

2. Utilisation selon la revendication 1, dans laquelle le fragment angiostatique de TrpRS est le T2-TrpRS (SEQ ID NO: 1).

3. Utilisation selon la revendication 1, dans laquelle le fragment angiostatique de TrpRS est le T2-TrpRS-GD (SEQ ID NO: 2).

4. Utilisation selon la revendication 1, dans laquelle le fragment angiostatique de TrpRS est le mini-TrpRS (SEQ ID NO: 3).

5. Utilisation selon la revendication 1, dans laquelle le fragment angiostatique de TrpRS est le T1-TrpRS (SEQ ID NO: 4).


### SEQUENCE LISTING

<110> THE SCRIPPS RESEARCH INSTITUTE
<120> REVASCULARIZATION OF ISCHEMIC RETINAL
   TISSUE
<130> 18-215 T1
<140> EP
   <141> 2006-02-24
<150> 60/655,732
   <151> 2005-02-24
<160> 4
<170> FastSEQ for Windows version 4.0
<210> 1
   <211> 378
   <212> PRT
   <213> Homo sapiens
<400> 1
   Ser Ala Lys Gly Ile Asp Tyr Asp Lys Leu Ile Val Arg Phe Gly Ser
   1               5                   10                  15
   Ser Lys Ile Asp Lys Glu Leu Ile Asn Arg Ile Glu Arg Ala Thr Gly
               20                  25                  30
   Gln Arg Pro His His Phe Leu Arg Arg Gly Ile Phe Phe Ser His Arg
           35                  40                  45
   Asp Met Asn Gln Val Leu Asp Ala Tyr Glu Asn Lys Lys Pro Phe Tyr
       50                  55                  60
   Leu Tyr Thr Gly Arg Gly Pro Ser Ser Glu Ala Met His Val Gly His
   65                  70                  75                  80
   Leu Ile Pro Phe Ile Phe Thr Lys Trp Leu Gln Asp Val Phe Asn Val
                   85                  90                  95
   Pro Leu Val Ile Gln Met Thr Asp Asp Glu Lys Tyr Leu Trp Lys Asp
               100                 105                 110
   Leu Thr Leu Asp Gln Ala Tyr Ser Tyr Ala Val Glu Asn Ala Lys Asp
           115                 120                 125
   Ile Ile Ala Cys Gly Phe Asp Ile Asn Lys Thr Phe Ile Phe Ser Asp
       130                 135                 140
   Leu Asp Tyr Met Gly Met Ser Ser Gly Phe Tyr Lys Asn Val Val Lys
   145                 150                 155                 160
   Ile Gln Lys His Val Thr Phe Asn Gln Val Lys Gly Ile Phe Gly Phe
                   165                 170                 175
   Thr Asp Ser Asp Cys Ile Gly Lys Ile Ser Phe Pro Ala Ile Gln Ala
               180                 185                 190
   Ala Pro Ser Phe Ser Asn Ser Phe Pro Gln Ile Phe Arg Asp Arg Thr
           195                 200                 205
   Asp Ile Gln Cys Leu Ile Pro Cys Ala Ile Asp Gln Asp Pro Tyr Phe
       210                 215                 220
   Arg Met Thr Arg Asp Val Ala Pro Arg Ile Gly Tyr Pro Lys Pro Ala
   225                 230                 235                 240
   Leu Leu His Ser Thr Phe Phe Pro Ala Leu Gln Gly Ala Gln Thr Lys
                   245                 250                 255
   Met Ser Ala Ser Asp Pro Asn Ser Ser Ile Phe Leu Thr Asp Thr Ala
               260                 265                 270
   Lys Gln Ile Lys Thr Lys Val Asn Lys His Ala Phe Ser Gly Gly Arg
           275                 280                 285
   Asp Thr Ile Glu Glu His Arg Gln Phe Gly Gly Asn Cys Asp Val Asp
       290                 295                 300
   Val Ser Phe Met Tyr Leu Thr Phe Phe Leu Glu Asp Asp Asp Lys Leu
   305                 310                 315                 320
   Glu Gln Ile Arg Lys Asp Tyr Thr Ser Gly Ala Met Leu Thr Gly Glu
                   325                 330                 335
   Leu Lys Lys Ala Leu Ile Glu Val Leu Gln Pro Leu Ile Ala Glu His
               340                 345                 350
   Gln Ala Arg Arg Lys Glu Val Thr Asp Glu Ile Val Lys Glu Phe Met
           355                 360                 365
   Thr Pro Arg Lys Leu Ser Phe Asp Phe Gln
       370                 375
<210> 2
   <211> 378
   <212> PRT
   <213> Homo sapiens
<400> 2
   Ser Ala Lys Gly Ile Asp Tyr Asp Lys Leu Ile Val Arg Phe Gly Ser
   1 5 10 15
   Ser Lys Ile Asp Lys Glu Leu Ile Asn Arg Ile Glu Arg Ala Thr Gly
   20 25 30
   Gln Arg Pro His His Phe Leu Arg Arg Gly Ile Phe Phe Ser His Arg
   35 40 45
   Asp Met Asn Gln Val Leu Asp Ala Tyr Glu Asn Lys Lys Pro Phe Tyr
   50 55 60
   Leu Tyr Thr Gly Arg Gly Pro Ser Ser Glu Ala Met His Val Gly His
   65 70 75 80
   Leu Ile Pro Phe Ile Phe Thr Lys Trp Leu Gln Asp Val Phe Asn Val
   85 90 95
   Pro Leu Val Ile Gln Met Thr Asp Asp Glu Lys Tyr Leu Trp Lys Asp
   100 105 110
   Leu Thr Leu Asp Gln Ala Tyr Gly Asp Ala Val Glu Asn Ala Lys Asp
   115 120 125
   Ile Ile Ala Cys Gly Phe Asp Ile Asn Lys Thr Phe Ile Phe Ser Asp
   130 135 140
   Leu Asp Tyr Met Gly Met Ser Ser Gly Phe Tyr Lys Asn Val Val Lys
   145 150 155 160
   Ile Gln Lys His Val Thr Phe Asn Gln Val Lys Gly Ile Phe Gly Phe
   165 170 175
   Thr Asp Ser Asp Cys Ile Gly Lys Ile Ser Phe Pro Ala Ile Gln Ala
   180 185 190
   Ala Pro Ser Phe Ser Asn Ser Phe Pro Gln Ile Phe Arg Asp Arg Thr
   195 200 205
   Asp Ile Gln Cys Leu Ile Pro Cys Ala Ile Asp Gln Asp Pro Tyr Phe
   210 215 220
   Arg Met Thr Arg Asp Val Ala Pro Arg Ile Gly Tyr Pro Lys Pro Ala
   225 230 235 240
   Leu Leu His Ser Thr Phe Phe Pro Ala Leu Gln Gly Ala Gln Thr Lys
   245 250 255
   Met Ser Ala Ser Asp Pro Asn Ser Ser Ile Phe Leu Thr Asp Thr Ala
   260 265 270
   Lys Gln Ile Lys Thr Lys Val Asn Lys His Ala Phe Ser Gly Gly Arg
   275 280 285
   Asp Thr Ile Glu Glu His Arg Gln Phe Gly Gly Asn Cys Asp Val Asp
   290 295 300
   Val Ser Phe Met Tyr Leu Thr Phe Phe Leu Glu Asp Asp Asp Lys Leu
   305 310 315 320
   Glu Gln Ile Arg Lys Asp Tyr Thr Ser Gly Ala Met Leu Thr Gly Glu
   325 330 335
   Leu Lys Lys Ala Leu Ile Glu Val Leu Gln Pro Leu Ile Ala Glu His
   340 345 350
   Gln Ala Arg Arg Lys Glu Val Thr Asp Glu Ile Val Lys Glu Phe Met
   355 360 365
   Thr Pro Arg Lys Leu Ser Phe Asp Phe Gln
   370 375
<210> 3
   <211> 423
   <212> PRT
   <213> Homo sapiens
<400> 3
   Ser Tyr Lys Ala Ala Ala Gly Glu Asp Tyr Lys Ala Asp Cys Pro Pro
   1               5                   10                  15
   Gly Asn Pro Ala Pro Thr Ser Asn His Gly Pro Asp Ala Thr Glu Ala
               20                  25                  30
   Glu Glu Asp Phe Val Asp Pro Trp Thr Val Gln Thr Ser Ser Ala Lys
           35                  40                  45
   Gly Ile Asp Tyr Asp Lys Leu Ile Val Arg Phe Gly Ser Ser Lys Ile
       50                  55                  60
   Asp Lys Glu Leu Ile Asn Arg Ile Glu Arg Ala Thr Gly Gln Arg Pro
   65                  70                  75                  80
   His His Phe Leu Arg Arg Gly Ile Phe Phe Ser His Arg Asp Met Asn
                   85                  90                  95
   Gln Val Leu Asp Ala Tyr Glu Asn Lys Lys Pro Phe Tyr Leu Tyr Thr
               100                 105                 110
   Gly Arg Gly Pro Ser Ser Glu Ala Met His Val Gly His Leu Ile Pro
           115                 120                 125
   Phe Ile Phe Thr Lys Trp Leu Gln Asp Val Phe Asn Val Pro Leu Val
       130                 135                 140
   Ile Gln Met Thr Asp Asp Glu Lys Tyr Leu Trp Lys Asp Leu Thr Leu
   145                 150                 155                 160
   Asp Gln Ala Tyr Ser Tyr Ala Val Glu Asn Ala Lys Asp Ile Ile Ala
                   165                 170                 175
   Cys Gly Phe Asp Ile Asn Lys Thr Phe Ile Phe Ser Asp Leu Asp Tyr
               180                 185                 190
   Met Gly Met Ser Ser Gly Phe Tyr Lys Asn Val Val Lys Ile Gln Lys
           195                 200                 205
   His Val Thr Phe Asn Gln Val Lys Gly Ile Phe Gly Phe Thr Asp Ser
       210                 215                 220
   Asp Cys Ile Gly Lys Ile Ser Phe Pro Ala Ile Gln Ala Ala Pro Ser
   225                 230                 235                 240
   Phe Ser Asn Ser Phe Pro Gln Ile Phe Arg Asp Arg Thr Asp Ile Gln
                   245                 250                 255
   Cys Leu Ile Pro Cys Ala Ile Asp Gln Asp Pro Tyr Phe Arg Met Thr
               260                 265                 270
   Arg Asp Val Ala Pro Arg Ile Gly Tyr Pro Lys Pro Ala Leu Leu His
           275                 280                 285
   Ser Thr Phe Phe Pro Ala Leu Gln Gly Ala Gln Thr Lys Met Ser Ala
       290                 295                 300
   Ser Asp Pro Asn Ser Ser Ile Phe Leu Thr Asp Thr Ala Lys Gln Ile
   305                 310                 315                 320
   Lys Thr Lys Val Asn Lys His Ala Phe Ser Gly Gly Arg Asp Thr Ile
                   325                 330                 335
   Glu Glu His Arg Gln Phe Gly Gly Asn Cys Asp Val Asp Val Ser Phe
               340                 345                 350
   Met Tyr Leu Thr Phe Phe Leu Glu Asp Asp Asp Lys Leu Glu Gln Ile
           355                 360                 365
   Arg Lys Asp Tyr Thr Ser Gly Ala Met Leu Thr Gly Glu Leu Lys Lys
       370                 375                 380
   Ala Leu Ile Glu Val Leu Gln Pro Leu Ile Ala Glu His Gln Ala Arg
   385                 390                 395                 400
   Arg Lys Glu Val Thr Asp Glu Ile Val Lys Glu Phe Met Thr Pro Arg
                   405                 410                 415
   Lys Leu Ser Phe Asp Phe Gln
               420
<210> 4
   <211> 401
   <212> PRT
   <213> Homo sapiens
<400> 4
   Ser Asn His Gly Pro Asp Ala Thr Glu Ala Glu Glu Asp Phe Val Asp
   1               5                   10                  15
   Pro Trp Thr Val Gln Thr Ser Ser Ala Lys Gly Ile Asp Tyr Asp Lys
                   20                          25                          30
   Leu Ile Val Arg Phe Gly Ser Ser Lys Ile Asp Lys Glu Leu Ile Asn
               35                          40                          45
   Arg Ile Glu Arg Ala Thr Gly Gln Arg Pro His His Phe Leu Arg Arg
       50                          55                          60
   Gly Ile Phe Phe Ser His Arg Asp Met Asn Gln Val Leu Asp Ala Tyr
   65                      70                          75                          80
   Glu Asn Lys Lys Pro Phe Tyr Leu Tyr Thr Gly Arg Gly Pro Ser Ser
                   85                          90                          95
   Glu Ala Met His Val Gly His Leu Ile Pro Phe Ile Phe Thr Lys Trp
               100                         105                         110
   Leu Gln Asp Val Phe Asn Val Pro Leu Val Ile Gln Met Thr Asp Asp
           115                         120                         125
   Glu Lys Tyr Leu Trp Lys Asp Leu Thr Leu Asp Gln Ala Tyr Ser Tyr
       130                         135                         140
   Ala Val Glu Asn Ala Lys Asp Ile Ile Ala Cys Gly Phe Asp Ile Asn
   145                     150                         155                         160
   Lys Thr Phe Ile Phe Ser Asp Leu Asp Tyr Met Gly Met Ser Ser Gly
                   165                         170                         175
   Phe Tyr Lys Asn Val Val Lys Ile Gln Lys His Val Thr Phe Asn Gln
               180                         185                         190
   Val Lys Gly Ile Phe Gly Phe Thr Asp Ser Asp Cys Ile Gly Lys Ile
           195                         200                         205
   Ser Phe Pro Ala Ile Gln Ala Ala Pro Ser Phe Ser Asn Ser Phe Pro
       210                         215                         220
   Gln Ile Phe Arg Asp Arg Thr Asp Ile Gln Cys Leu Ile Pro Cys Ala
   225                     230                         235                         240
   Ile Asp Gln Asp Pro Tyr Phe Arg Met Thr Arg Asp Val Ala Pro Arg
                   245                         250                         255
   Ile Gly Tyr Pro Lys Pro Ala Leu Leu His Ser Thr Phe Phe Pro Ala
               260                         265                         270
   Leu Gln Gly Ala Gln Thr Lys Met Ser Ala Ser Asp Pro Asn Ser Ser
           275                         280                         285
   Ile Phe Leu Thr Asp Thr Ala Lys Gln Ile Lys Thr Lys Val Asn Lys
       290                         295                         300
   His Ala Phe Ser Gly Gly Arg Asp Thr Ile Glu Glu His Arg Gln Phe
   305                     310                         315                         320
   Gly Gly Asn Cys Asp Val Asp Val Ser Phe Met Tyr Leu Thr Phe Phe
                   325                         330                         335
   Leu Glu Asp Asp Asp Lys Leu Glu Gln Ile Arg Lys Asp Tyr Thr Ser
               340                         345                         350
   Gly Ala Met Leu Thr Gly Glu Leu Lys Lys Ala Leu Ile Glu Val Leu
           355                         360                         365
   Gln Pro Leu Ile Ala Glu His Gln Ala Arg Arg Lys Glu Val Thr Asp
       370                         375                         380
   Glu Ile Val Lys Glu Phe Met Thr Pro Arg Lys Leu Ser Phe Asp Phe
   385                     390                         395                         400
   Gln